Europäisches Patentamt

European Patent Office

Office européen des brevets

(19)

(11) Publication number: **0 111 344**
A2

(12)

# EUROPEAN PATENT APPLICATION

(21) Application number: **83112532.3**

(22) Date of filing: **13.12.83**

(51) Int. Cl.³: **C 12 P 21/00**
**C 12 N 5/00, C 12 N 15/00**
**G 01 N 33/54, G 01 N 33/56**
**A 61 K 39/395**

(30) Priority: **13.12.82 US 449329**

(43) Date of publication of application:
**20.06.84 Bulletin 84/25**

(84) Designated Contracting States:
**AT BE CH DE FR GB IT LI LU NL SE**

(71) Applicant: **Sloan-Kettering Institute For Cancer Research**
**1275 York Avenue**
**New York New York 10021(US)**

(72) Inventor: **Feickert, Hans-Joachim**
**544 East 81 Street**
**New York New York(US)**

(72) Inventor: **Welte, Karl**
**504 East 81 Street**
**New York New York(US)**

(72) Inventor: **Mertelsmann, Roland**
**301 Millwood Road**
**Chappaqua New York 10514(US)**

(74) Representative: **Patentanwälte Schulze Horn und Hoffmeister**
**Goldstrasse 36**
**D-4400 Münster(DE)**

(54) **Anti-interleukin-2 monoclonal antibodies.**

(57) Monoclonal antibodies recognizing interleukin-2 (IL-2) of various species (human, rat, mouse) are disclosed. These antibodies are useful in purification of IL-2 from biological sample as well as in separating IL-2 into various sub-species. Immunoassay of IL-2 and identification of cells containing IL-2 are made possible by these monoclonal antibodies. The monoclonal antibodies are also of therapeutic use in the treatment of syndromes associated with hyperproduction of interleukin-2 including hyperimmune syndromes GvH, acute lymphoblastic leukemia, T-cell lymphoma or leukemia.

EP 0 111 344 A2

Croydon Printing Company Ltd.

## Anti-Interleukin 2-Monoclonal Antibodies

This invention concerns monoclonal antibodies which recognize interleukin 2 (IL-2) a cytokine important in the regulation of immune function. Immunoassay of IL-2 and thereapy of immuno-deficiency diseases are made possible by these monoclonal antibodies.

## INTRODUCTION

The regulation of immune function (Paetkau, V., Nature (Lond.) 294 689 (1981)) and tumor growth (Sparn, M. B. and Todaro, G. J., N. Engl. J. med. 303, 878 (1980)) by hormone like factors, cytokines, has become the subject of increasing interest. One such cytokine, Interleukin 2 (IL-2) has been discovered (Morgan, D. A. et.al., Science 193, 1007 (1976)). It is produced by T lymphocytes after antigen or mitogen stimulation and is required for the proliferation of activated T cells. IL2 is an essential mediator of the immune response (Ruscetti, F. W. and Gallo, R. C., Blood 57, 379 (1981); Smith, K. A., Immunol. Rev., 51 337 (1980)). It plays a central role in the cellular immune response to foreign antigens as well as in the development or sustaining of certain lymphoid leukemias (Poiesz, B. J. et al., Proc. Natl. Acad. Science, 77 6815 (1980)). There is preliminary evidence that IL2 may also be responsible for the clonal growth of human lymphblastic leukemias (Venuta, S. et al. Blood (1982)). Furthermore it has been demonstrated that the mitogenic response of lymphocytes from patients with severe immunodeficiencies can be restored in vitro by adding highly purified IL-2 to the tissue cultures. To date there is some indication that pure human IL-2 might restore some immunfunctions even in vivo as demonstrated in a first clinical trial in a patient with severe combined immunodeficiency (Nezelof-Syndrom).

Studies on the physiology and pathaphysiology of IL2 are dependent on the availability of a well defined, biochemically and biologically homogenous molecule. IL2 has been purified to apparent homogeneity (Welte, K. et al. J. Exp. Med. 1982) in order to study its role as mediator of the normal immune response, in human immunodeficiency syndromes and in acute lymphblastic leukemias (ALL).

However, further elucidation of the biological significance of this important cytokine await further purification.

## SUMMARY OF THE INVENTION

IL2 purified by known methods (Welte, et al. supra) was used to produce a mouse monoclonal antibody against IL2 by the technique of Köhler-Milstein. The fusion resulted in hybrid clones producing anti-IL-2 of various subclasses (IgA, IgG-2b, IgM). The hybrid clone secreting anti-IL-2 antibody of subclass IgA was selected as preferred embodiment of the present invention. All anti-IL-2 antibodies inhibited the proliferation of IL-2 dependent human and mouse cell lines in response to human highly purified IL-2. One of these antibodies chosen for further characterization selectively precipitated $14K^{125}I$-IL-2 as

well as $16K^{125}I$-IL-2 and $17K^{125}I$-IL-2.

The anti-IL2 antibodies are useful in the purification of IL-2 in the establishment of screening assays for IL-2, immunoassays of IL-2, radioimmunoassay and enzyme-linked immunoassay, for example, (ELISA, RIA) and for dissection of the role of IL-2 in the immune system. Furthermore these monoclonal antibodies facilitate the investigation of the previously observed heterogeneity of IL-2.

The anti-IL2 monoclonal antibodies of the present invention are also useful as therapeutic agents in clinical syndromes which are associated with pathological proliferation of IL-2 dependent cells. Thus, for example, cutaneous T-cell lymphomas, acute lymphoblastic leukemias (ALL) and hyperimmune syndromes such as Graft versus Host disease (GvH) may be treated. In a preferred embodiment of the present invention, the anti-IL-2 monoclonal antibodies are used as therapeutic agents directly without further modification thereof. Alternatively, the antibodies may be coupled to cytotoxic agents which are capable of blocking IL-2-dependent cell divisions. The monoclonal antibodies of the present invention are capable of recognizing specifically cells expressing IL-2 receptors where the coupled cytotoxic agents exert there effect on blocking IL-2-dependent cell divisions.

The monoclonal antibodies of the present invention are also useful diagnostic reagents for cells which contain IL-2 either on the cell surface or within the cytoplasm of the cells. Thus by means of the present invention, cells containing IL-2 may be identified in samples having different kinds of cells. Localization of IL-2-containing cells is possible in cultured cell colonies or in tissue specimens. When used in this

4

manner the monoclonal antibodies are preferably coupled to flourescent, color-forming substance such as an enzyme or chromophor, or a radioactive substance.

DETAILED DESCRIPTION OF THE INVENTION

The following description is intended to illustrate this invention without limiting same in any manner especially with respect to substantially functional equivalents hybridomas, monoclonal antibodies and cell lines described herein.

Availability of cell lines, hybridomas and monoclonal antibodies

The cell lines, hybridomas and monoclonal antibodies disclosed in the present invention bear the designated deposit number and are deposited with American Type Culture Collection, Rockville, Maryland, U S A. Deposit is for the purpose of enabling disclosure only and is not intended to limit the concept of the present invention to the particular materials deposited. The file number of the culture deposit is: HB 8456 I. Production of mAb

Source of IL-2

IL-2 was purified from lymphocyte conditioned medium to apparent homogeneity as described earlier (copending U. S. Patent Application No. 370,223). As judged by silver staining of sodium-dodecyl-sulfate-polyacrylamide gel electrophoresis (SDS-PAGE), IL-2 consists of 3 bands corresponding to molecular weights of 14,500, 16,000 and 17,000 daltons. The proteins eluted from each of those 3 bands showed IL-2 activity. Assay for IL-2 was detected by assay reported previously. (Welte, K., et al. supra).

5

The purity of the IL-2 used for the immunization is an
important criterion. For all injections material was
applied consisting of predominantly the 16K and 17K IL-2.
Both the 16K and 17K molecular weight IL-2 species
exhibit biological activity.

## Immunization

A 8 week old BALB/c x C57B6 female mouse was immunized
six times (in two weekly intervals) with 1 ml of highly
purified IL-2 (600-800 U/ml) by i. p injections. 5 days
after the last immunization the spleen of the mouse was
removed and used for the fusion.

## Fusion

Approximately 100 x $10^6$ immuno-splenocytes were fused
to 40 x $10^6$ NS-1-mouse-myeloma cells according to the
method of Koehler and Milstein. Three weeks after the
fusion outgrowing clones were tested for immunoglobulin
production by enzyme-linked immunoassay (ELISA) technique
and the specificity of positive clones established by a
neutralization assay for IL-2. Clones producing specific
antibodies were subcloned four times and either expanded as mass cultures or grown in nude mice or as
ascites in syngeneic mice.

## Antibody detection assay

After fusion, the growing clones were screened for
antibody production by enzyme-linked immunoassay (ELISA).
10ul culture supernatant was bound to a Terasaki
plate (Falcon) overnight at 4° C. Then a second antibody
(rabbit anti-mouse-IG-serum) at a dilution of 1 : 500
was added for 45 minutes at 37° C. After washing, 10ul
of goat-anti-rabbit-alkaline phosphate conjugate
(diluted 1 : 500) was added for 45 minutes at 37° C.

Finally, after 30 - 45 minutes incubation with a substrate (P-nitrophenyl phosphate (PNp) at 37° C, the plates were read by an ELISA reader with print-out of the measured absorbance.

Purification of anti-IL-2 antibody

A clone (designated number FW-4), producing anti IL-2 antibodies (designated number monoclonal antibody FW-4), was propagated in nude mice (Swiss background) and/or as mass tissue culture. Culture supernatants, containing 4 % immunoglobulin-free fetal calf serum (FCS) or mouse-sera were passed over a CM-Affi gel Blue column and the fall-through fractions collected. Proteases and the bulk of albumin remained bound to the column. The anti-IL-2 antibody containing fall-through fractions were concentrated by ammonium sulfate-precipitation, dialyzed against the appropriate buffer (Phosphate buffered saline (PBS) or tris-HCL, pH 7.8) and loaded either on an ion exchange chromatography column or on a gel filtration column. Anti-IL-2 eluted from the ion exchange chromatography in the unbound protein fractions and in the gel filtration chromatography was collected in fractions corresponding to MW of 150,000 - 170,000 daltons. The anti-IL-2 antibody-containing fractions were stored in aliquots at -70° C.

The fusion of spleen cells from mice immunized with highly purified human IL-2 with NS-1-myeloma cells resulted in the outgrowth of 107 clones of which 71 clones produced immunoglobulins detected by the ELISA technique. 5 of 25 clones tested for neutralization of IL-2 activity showed an inhibition of IL-2 activity. These clones produced immunoglobulin of the IgM, IgG 2b and IgA subclasses, respectively, as determined by Ouchterlony technique. The IgA secreting clone, FW4, was chosen for all further experiments. It is a stable, fast growing hybrid after four subcloning cycles by limiting dilution technique. The immunoglobulin sub-class of the Ig producing clone was determined by testing tissue culture supernatant with mouse-IG-heavy chain specific reagents by Ouchterlony technique.

Supernatants from the IgA secreting hybrid, grown in 4 % immunoglobulin free FCS was collected and purified by chromatography on CM-Affi gel blue and gel filtration on AcA 34 Ultrogel. The procedure resulted in removal of the bulk of unspecific proteins including proteases. After the final gel filtration step high neutralizing activity was recovered from fractions 39-41 corresponding to MW of 150,000 - 170,000 daltons.

II. Biochemical characterization of anti-IL-2 antibody, FW-4 Neutralization assay

(a) The effect of anti-IL-2 on T-cell mitogen-induced proliferation was tested. Ficol-hypaque separated mononuclear blood cells from normal donors were resuspended at $4 \times 10^6$ cells/ml in RPMI 1640 supplemented with 10 % heat inactivated fetal calf serum (FCS) and glutamine (2mM). Each sample was stimulated in triplicate microwell culture with either medium alone or Phytohemagglutinin (0.5 %, PHA-M). All stimulation assays were done in the presence or absence of 100 ul antibody solution (anti-IL-2 or control). The cultures were pulsed at day 3 with tritiated thymidine ($^3$H-dT, 0.5 uCi/microplate well, specific activity, 20 mCi/mM) and the incorporation of $^3$H-dT measured. Significant inhibition was observed with 1 ug anti-IL-2 per 1 ng1ml IL-2.

Anti-IL-2 mAb FW-4 also inhibited in a dose dependent manner the proliferation of T cell lines dependent on IL-2:

(a) 3 - 4 week cultures of human PHA stimulated lymphocytes,

(b) Mouse cytotoxic T cell lines CTLL-1 and A2. As shown in Table 1, the proliferation was inhibited even in culture medium containing IL-2 concentrations as high as 10 U/ml and 100 U/ml IL-2, respectively. These data suggest that anti-IL-2 (mAb FW-4) reacts with the biologically active site of IL-2.

## Table 1

Dose dependent inhibition of the IL-2 effect on IL-2 dependent cell lines by mAb FW-4

| | mAb FW-4 (ng/ml) | | | | |
|---|---|---|---|---|---|
| IL-2(ng/ml) | 1000 | 500 | 25 | 125 | 62.5 |
| 10 | 100 | 88 | 58 | 21 | 11 |
| 50 | 96 | 70 | 42 | 19 | 8 |
| 100 | 94 | 70 | 31 | 9 | 0 |

(b) Inhibition of growth of human and mouse CTLL

This specificity of mAb FW-4 was established by demonstrating the dose dependent inhibition of pure IL-2 on IL-2 dependent mouse T cell lines CTLL-1, and A-2, and PHA-induced normal human lymphocytes after three weeks of IL-2 dependent culture. Proliferation of human CTLL, mouse CTLL cells in the presence of variable amounts of monoclonal antibodies against IL-2 was compared to the IL-2 standard curve obtained in the absence of anti-IL-2 antibodies to determine the percentage of inhibition of IL-2 activity. The proliferation was inhibited even in culture medium containing 10 U/ml and 100 U/ml IL-2, respectively. These data suggest that anti-IL-2 reacts with the biologically active site of IL-2.

Species Specificity

IL-2 from various species (mouse, rat, human) were inhibited in the same amount in support of growth of mouse CTLL.

Immunoglobulin Sub-class

The hybrids produce mAbs of the IgM IgG$_{2b}$ and IgA immunoglobulin subclasses as determined by Ouchterlony technique. Since monoclonal antibodies of IgA subclass are rarely generated, this preferred clone was characterized in more detail. This IgA monoclonal antibody should be useful for employing double-labeling techniques for immunofluorescent analyses of IL-2 producer and responder cells. The IgA-nature of this antibody was supported by data received from the purification of culture supernatants (free of fetal calf immunoglobulin) which demonstrated that the mAb FW-4 has an approximate molecular weight of 160,000 daltons as determined by gel filtration. Further evidence comes from analysis of metabolically labeled ($^{35}$S-methionine) mAb FW-4 on SDS-PAGE under reducing conditions showing one heavy chain with approximate molecular weight of 55,000 daltons and two light chains with molecular weight of 22,000 and 24,000 daltons. One light chain is the kappa light chain produced by the parental myeloma line, the second light chain is probably of the lamda type (although we were not able to prove this by the Ouchterlony technique) and derived from the B-cell fusion partner of the hybrid.

## Immunoprecipitation procedure and following SDS-PAGE

### (a) highly purified IL-2

The IL-2 specificity of mAb FW-4 was further supported by the immunoprecipitation studies. Using highly purified IL-2 consisting predominantly of 14K and 17K molecular weights, stabilized with BSA, IL-2 was precipitated but not albumine.

Highly purified IL-2 consisting of only the 14K and 17K species was labeled with $^{125}$Iodine (Lacto-peroxidase technique). Traces of albumin present in the buffer for stabilization after the iodinization were labeled incidentically. Using the anti-IL-2 antibody (fraction 39-41 of AcA 34, corresponding to 160 K MW) and fraction 44-46 (corresponding to 130 K MW) as control, the 14K and 17K IL-2 were precipitated specifically. In the control, no IL-2 was precipitated.

### (b) Partially purified IL-2

By using partially purified IL-2 for immunoprecipitation, it was found that the anti-IL-2 antibody detects besides the IL-2 species between 14K and 17K, protein between 26K and 32K. Both the 26K and 32K IL-2 were present in some purification procedures depending on induction conditions and copurified with the 14K, 16K and 17K IL-2 until the last step of purification.

Highly purified IL-2 as well as partially purified IL-2 was iodinated ($^{125}$I) by the lactoperoxidase method by methods known in the art. 100 ul of the labeled protein solution (ca. 400,000 cpm) were incubated with 200 ul antibody solution (previously purified by Ion-exchange chromatography (DEAE-cellulose) and/or gel filtration on AcA 34 ultrogel) and subsequently with 50 ul rabbit-anti-mouse-Ig-serum for 16 hours at $4^{\circ}$ C. Then Staphylococcus Protein A was added for one hour at room temperature and the immune complexes were washed and analyzed in a SDS-PAGE (7 15 % acrylamide gradient) under reducing conditions (Laemmli, U. K., et al. Nature $\underline{277}$ 680 (1970)). As control material, eluated from the AcA 34 column in the 100,000 molecular weight range (anti-IL-2 purification step) and antibodies against lung tumor cell surface antigens or supernatants from NS-1 myeloma cell cultures, which showed no inhibition in the neutralization assay for IL-2, were treated in the same way. $^{125}$I-IL-2 was precipitated specifically by mAb FW-4, while in the control only background staining was observed.

A1

What is claimed is:

1. Monoclonal antibodies recognizing IL2.

2. The monoclonal antibody of Claim 1 of immunoglobulin sub-class A.

3. Antibody-producing hybridoma cell line characterized by the production of the antibodies as claimed in Claim 1.

4. Antibody-producing hybridoma cell line of Claim 2 formed by fusing a myeloma derived cell strain with splenocytes derived from BALB/C mice immunized with interleukin-2.

5. Antibody-producing hybridoma cell line of Claim 4 wherein said human interleukin-2 comprises fractions of molecular weight between about 14K and 17K.

6. Monoclonal antibody FW-4 having a molecular weight of about 160,000 daltons.

7. Method of purifying interleukin-2 from a sample comprising contacting said sample with monoclonal antibody of Claim 1 under conditions suitable for forming an interleukin 2-anti-interleukin-2 antibody complex; separating said complex from said sample; and treating said complex to separate interleukin-2 therefrom.

8. Method of separating sub-species of interleukin-2 from non-homogeneous mixtures of interleukin-2 comprising contacting said mixtures with monoclonal antibody of Claim 1 under conditions suitable for reaction.

9. Immunoassay of interleukin-2 in a sample comprising contacting said sample with the monoclonal antibody of Claim 1.

10. Immunoassay of Claim 9 wherein said immunoassay is radioimmunoassay.

11. Immunoassay of Claim 9 wherein said immunoassay is enzyme-linked immunoassay.

12. A method of inhibiting proliferation of interleukin-2 dependent cultured human and mouse cell lines comprising contacting said cultured cell lines with the monoclonal antibody of Claim 1.

13. Method of treating an individual having a syndrome associated with hyperproduction of interleukin-2 comprising administering anti-interleukin-1 monoclonal antibodies to said individual.

14. Method of Claim 13 wherein said monoclonal antibodies are coupled to a cytotoxic agent.

15. Method of Claim 13 wherein said syndrome is a hyperimmune syndrome.

16. Method of Claim 15 wherein said hyperimmune syndrome is GvH, acute lymphoblastic leukemia, T-cell lymphoma or leukemia.

17. Method for locating cells which contain interleukin-2 in a mixture of cells comprising immunoassay of said mixture with the monoclonal antibodies of Claim 1 and observing the reaction site.

18. The assay of Claim 17 wherein said monoclonal antibody is coupled to a fluorescent, chromophoric or a radioactive substance.